# EUROPEAN PATENT APPLICATION

(11) **EP 1 918 017 A1**
(43) Date of publication of application: **07.05.2008**
(21) Application number: 07104894.6
(22) Date of filing: 26.03.2007
(51) Int. Cl.: B01D 61/42, C12Q 1/68

(54) **Electrodialysis Apparatus and Electrodialysis Method Using the Same**

(30) Priority: 26.09.2006 KR 20060093715
(71) Applicant: Samsung Electronics Co., Ltd., Suwon-si, Gyeonggi-Do (KR)
(72) Inventor: Lee, Hun-joo c/o Samsung Advanced Inst. of Technology, Gyeonggi-do (KR); Lee, Soo-suk c/o Samsung Advanced Inst. of Technology, Gyeonggi-do (KR); Kim, Joon-ho c/o Samsung Advanced Inst. of Technology, Gyeonggi-do (KR); Jung, Sung-ouk c/o Samsung Advanced Inst. of Technology, Gyeonggi-do (KR); Lee, In-ho c/o Samsung Advanced Inst. of Technology, Gyeonggi-do (KR)
(74) Representative: Grünecker, Kinkeldey, Stockmair & Schwanhäusser Anwaltssozietät

(57) **Abstract**

Provided are an electrodialysis apparatus (1) for preparing PCR samples and an electrodialysis method using the same. The electrodialysis apparatus (1) comprises an insertable sample chamber (100); a first dialysis membrane (110) and a second dialysis membrane (120); an anode chamber (210) including an anode (211), wherein the first dialysis membrane (110) separates the anode chamber (210) and the sample chamber (100); and a cathode chamber (220) including a cathode (221), wherein the second dialysis membrane (120) separates the cathode chamber (220) and the sample chamber (100). In particular, when a voltage is applied to the anode (211) and the cathode (221), ionic materials of the sample move towards the anode (211) and cathode (221). The electrode chambers can be agitated by means of supplied gas (262).

## Description

### BACKGROUND OF THE INVENTION

### 1. Field of the Invention

The present invention relates to an electrodialysis apparatus and an electrodialysis method using the same.

### 2. Description of the Related Art

A polymerase chain reaction (PCR) is generally an artificial gene amplification method. In brief, single-stranded deoxyribonucleic acid (DNA) molecules are obtained by separating DNA double helixes via heat treatment. The single strand DNA can be used as templates for a heat-stable DNA polymerase, which synthesizes new double helixes. More specifically, heating double-stranded DNA separates the double-stranded DNA into single-stranded DNA. Primers, which are short pieces of DNA, are added to the single-stranded DNA. Upon cooling, the primers hybridize with the single-stranded DNA. A DNA polymerase is added to the single DNA strands with the primers, and the DNA polymerase initiates DNA replication starting from the primers as the origin of replication. Repeating this cycle more than several tens of times allows DNA amplification up to more than several billions of DNA within a short period of time.

In a human body-related PCR, in general, a body fluid is used as PCR sample. However, PCR inhibitors are present within many body fluids, which inhibitors reduce the PCR efficiency to a great extent. Salt and red blood cells (RBCs) are representative examples of such a PCR inhibitor. For instance, exemplary materials comprising salt include sodium chloride (NaCl) and sodium sulphate (Na₂SO₄). Blood or urine, which may be used as PCR sample, contain a high content of salt or RBCs.

When DNA of bacteria, which cause septicemia, is to be amplified or detected from the blood of a patient associated with septicemia, it is usually necessary to separate this target bacterial DNA from the blood sample in order to effectively amplify or detect the target bacterial DNA. That is, the PCR inhibitors needs to be removed from the blood sample prior to PCR.

Direct use of the non-diluted whole blood as a PCR sample is limited because the non-diluted whole blood contains lots of PCR inhibitors. Generally, PCR efficiency decreases when about 2% of blood is contained within the total reaction solution.

One approach to overcome this limitation is a method of selectively destroying RBCs. For instance, U.S. patent application no. 4,407,942 issued to H. C. Birnboim on October 4, 1983, entitled "Fluorescent Detection of DNA Damage" teaches a method of selectively destroying RBCs using ammonium chloride. However, this conventional method of removing PCR inhibitors from a sample except for target DNA may be very complex and take a long time. Also, the sample is likely to be contaminated while separating the target DNA from the sample.

Accordingly, an apparatus and method that can effectively remove a PCR inhibitor need to be developed to allow a direct and simple PCR using the whole blood.

### SUMMARY OF THE INVENTION

The present invention provides an apparatus and a method that can improve the efficiency of a polymerase chain reaction (PCR), which often decreases due to a PCR inhibitor when a direct PCR is executed using the whole blood.

According to an aspect of the present invention, there is provided an electrodialysis apparatus, including a sample chamber including first and second dialysis membranes and filled with a sample between the first dialysis membrane and the second dialysis membrane, an anode chamber including an anode and filled with a chamber solution between the anode and the first dialysis membrane, and a cathode chamber including a cathode and filled with a chamber solution between the cathode and the second dialysis membrane, wherein when a voltage is applied to the anode and the cathode, ionic materials of the sample move to the anode and cathode chambers. That is, there is provided an electrodialysis apparatus comprising: a sample chamber; a first dialysis membrane and a second dialysis membrane; an anode chamber including an anode, wherein the first dialysis membrane separates the anode chamber and the sample chamber; and a cathode chamber including a cathode, wherein the second dialysis membrane separates the cathode chamber and the sample chamber.

In one embodiment of the present invention, the first dialysis membrane and the second dialysis membrane form wall of the sample chamber, said the first dialysis membrane and the second dialysis membrane are preferably positioned opposing each other. In one embodiment, the first dialysis membrane and the second dialysis membrane constitute, at least section wise, side wall areas of the sample chamber.

In one embodiment of the present invention, the first and second dialysis membranes may include a molecular weight cutoff membrane. The molecular weight cutoff membrane may include one selected from a cellophane membrane, a cellulose membrane, a PES (polyether sulfone) membrane, a PS (polysulfone) membrane, a PVDF (polyvinylidene fluoride) membrane, and a combination thereof.

In one embodiment of the present invention, the chamber solutions may include deionized water.

The electrodialysis apparatus may further include a control block (or control means) controlling an ionic concentration of each of the anode chamber and the cathode chamber to be lower than a reference ionic concentration. The control means of the electrolysis apparatus is capable of controlling an ionic concentration of a chamber solution that is present in the anode chamber and/or the cathode chamber to be lower than a reference ionic concentration.

In one embodiment of the present invention, the control block may replace the whole of the chamber solutions with new chamber solutions if the ionic concentration of the anode chamber or the cathode chamber is higher than the reference ionic concentration. The control means may comprise means for replacing the whole or a part of the chamber solutions in the anode chamber and/or the cathode chamber with new chamber solutions.

The reference ionic concentration may be lower than an ionic concentration of the sample chamber.

The control block may include a sensor unit sensing the ionic concentrations of the anode chamber, the cathode chamber and the sample chamber, and a pump supplying or discharging the chamber solutions. In other words, The control means may comprises a sensor unit for sensing the ionic concentration of the chamber solution in the anode chamber, the cathode chamber and/or a sample solution in the sample chamber; and a pump for supplying or discharging the chamber solutions.

The sensor unit may sense the ionic concentrations of the anode chamber, the cathode chamber and the sample chamber by measuring electrical resistance or electrical conductivity of each of the anode chamber, the cathode chamber and the sample chamber. In one embodiment, the sensor unit comprises an electrical resistance measuring means or an electrical conductivity measuring means capable of sensing the ionic concentrations of a chamber solution in the anode chamber, the cathode chamber and/or a sample solution in the sample chamber.

The electrodialysis apparatus may further include a mixing block (or mixing means) causing the chamber solutions of the respective anode and cathode chambers to flow, that is, the mixing means is capable of mixing the chamber solutions in the anode chamber and/or the cathode chamber.

The mixing block may generate bubbles inside the anode and cathode chambers. The mixing means may therefore comprise means capable of supplying bubbles into the anode chamber and/or the cathode chamber. The means capable of supplying bubbles may comprise at least one outlet through which, during operation, bubbles are released into the anode chamber and/or the cathode chamber, wherein the at least one outlet is positioned such that to the bubbles released through said outlet are capable of removing a dialysis interference layer formed at the interface between the first dialysis membrane and the anode chamber and/or between the second dialysis membrane and the cathode chamber.

The anode and the cathode may be spaced apart from each other by a range of minimum distance so as to allow smooth flux of the chamber solutions around the respective first and second dialysis membranes. The sample chamber may be formed in a tetragonal shape and/or may be attachable to or detachable from from the electrodialysis apparatus, preferable from a space between the anode chamber and the cathode chamber.

In one embodiment, the electrodialysis apparatus may further comprise a housing capable of being filled with a chamber solution, wherein the anode and the cathode are positioned spaced apart from each other inside the housing.

The sample chamber may be attachable to or detachable from the housing in a position between the anode and the cathode. In an assembled state of the electrodialysis apparatus the chamber solution in the anode chamber, the first dialysis membrane, the sample in the sample chamber, the second dialysis membrane, and the chamber solution in the cathode chamber are arranged in sequential order between the anode and the cathode.

According to another aspect of the present invention, there is provided an electrodialysis apparatus, including a housing filled with chamber solutions, an anode and a cathode spaced apart from each other inside the housing and generating an electrical field, and a sample chamber filled with a sample between dialysis membranes of the sample chamber and attachable to or detachable from the housing, wherein each of the dialysis membranes forms a wall isolating the corresponding chamber solution from the sample, and ionic materials contained in the sample move to the chamber solutions through the corresponding dialysis membranes due to the electric field. The dialysis membranes may include a molecular weight cutoff membrane. The chamber solutions may include deionized water.

In another embodiment of the present invention, the electrodialysis apparatus may further include a control block controlling ionic concentrations of the chamber solutions to be lower than a reference ionic concentration. The control block may replace the whole of one of the chamber solutions with a new chamber solution if one of the ionic concentrations of the chamber solutions is substantially the same as or higher than the reference ionic concentration. The reference ionic concentration may be lower than an ionic concentration of the sample. The control block may sense the ionic concentrations of the chamber solutions by measuring electrical resistance or electric conductivity of each of the chamber solutions and sample.

In another embodiment of the present invention, the electrodialysis apparatus may further include a mixing block generating bubbles to make the chamber solutions flow.

In another embodiment of the present invention, the sample chamber may be formed in a tetragonal shape, and the dialysis membranes may be formed on both sides of the sample chamber.

According to another embodiment of the present invention, there is provided an electrodialysis apparatus, including an anode and a cathode generating an electric field, first and second dialysis membranes, first and second chamber solutions, and a sample, wherein the first chamber solution, the first dialysis membrane, the sample, the second dialysis membrane, and the second chamber solution are arranged in sequential order in between the anode and the cathode; ionic materials contained in the sample are dialyzed into the first and second chamber solutions due to the electric field; and the first and second dialysis membranes include a molecular weight cutoff membrane.

In another embodiment of the present invention, the electrodialysis apparatus may further include a control block replacing the whole of the first or second chamber solution with a new chamber solution if the first or second chamber solution has an ionic concentration substantially the same as or higher than a reference ionic concentration.

In another embodiment of the present invention, the electrodialysis apparatus may further include a mixing block generating bubbles in order to remove dialysis interference layers formed at interfaces between the first dialysis membrane and the first chamber solution and between the second dialysis membrane and the second chamber solution.

In another embodiment of the present invention, the anode and the cathode may be spaced a minimum distance from each other.

According to a further another embodiment of the present invention, there is provided an electrodialysis method, including arranging a first chamber solution, a first dialysis membrane, a sample, a second dialysis membrane, and a second chamber solution in sequential order in between an anode and a cathode that are generating an electric field, and dialyzing ionic materials contained in the sample into the first and second chamber solutions based on the electric field, wherein the first and second dialysis membranes include a molecular weight cutoff membrane. In other words, the electrodialysis method comprises the steps of arranging a first chamber solution, a first dialysis membrane, a sample, a second dialysis membrane, and a second chamber solution in sequential order between an anode and a cathode that are generating an electric field; and dialyzing ionic materials contained in the sample through the respective dialysis membrane into the first and second chamber solutions based on the electric field.

In another embodiment of the present invention, the electrodialysis method of may further comprise the step of controlling the ionic concentration of the first chamber solution and/or the second chamber solution to be lower than a reference ionic concentration.

In another embodiment of the present invention, the electrodialysis method may further include replacing the whole or a part of the first chamber solution and/or the second chamber solution with a new one if the first and/or second chamber solution has an ionic concentration substantially the same as or higher than a reference ionic concentration. That is, a chamber solution is preferably replaced with new chamber solution if the ionic concentration is higher than the reference ionic concentration. In one embodiment the electrical resistance or electrical conductivity of the first chamber solution, the second chamber solution and/or the sample solution is measured in order to determine in the ionic concentration is greater than a reference concentration.

In another embodiment of the present invention, the electrodialysis method may comprise the step of mixing the first chamber solution and/or the second chamber solution. The mixing can be achieved by releasing bubbles into the first chamber solution and/or the second chamber solution, that is the first and second chamber solutions may flow due to bubbles.

In another embodiment of the present invention, the method may further comprise removing a dialysis interference layer formed at an interfaces between the first dialysis membrane and the first chamber solution and/or between the second dialysis membrane and the second chamber solution. The removing of a dialysis interference layer by carried out by means of bubbles.

In another embodiment of the present invention, the anode and the cathode may be spaced a minimum distance from each other in order to maximize the intensity of the electric field.

In another embodiment of the present invention, the sample may be filled between the first dialysis membrane and the second dialysis membrane, in the form of an integral cartridge type, and attachable to or detachable from a space between the first chamber solution and the second chamber solution. In other words, the electrodialysis method the sample may be filled between the first dialysis membrane and the second dialysis membrane of a sample chamber designed in the form of an integral cartridge type, and attaching the sample cartridge to a space between the first chamber solution and the second chamber solution; applying a voltage to the anode and the cathode and performing electrodialysis; and detaching the sample cartridge containing the electrodialysed sample from the space between the first chamber solution and the second chamber solution.

### BRIEF DESCRIPTION OF THE DRAWINGS

The above and other features and advantages of the present invention will become more apparent by describing in detail exemplary embodiments thereof with reference to the attached drawings in which:
FIG. 1 illustrates an exploded perspective view of an electrodialysis apparatus comprising a housing and an attachable and detachable sample chamber according to an embodiment of the present invention;
FIG. 2 is a sectional view of the electrodialysis apparatus illustrated in FIG. 1 in an assembled state, taken along a line I-I';
FIG. 3 is a sectional view of the electrodialysis apparatus illustrated in FIG. 1 in an assembled state, taken along a line II-II' illustrated in FIG. 1; and
FIG. 4 is a diagram illustrating the ion transfer from a sample into the chamber solutions occurring when performing electrodialysis in the electrodialysis apparatus.

### DETAILED DESCRIPTION OF THE INVENTION

The present invention will now be described in more detail with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those of ordinary skill in the art.

When a direct polymerase chain reaction (PCR) is performed without diluting a whole blood sample, lots of blood cells and salt, which exist in whole blood, usually act as PCR inhibitors, and thus, a PCR can be performed using only a low amount of blood. Since the whole blood is electrically dialyzed and used as a blood sample, however, according to an embodiment of the present invention, a direct PCR can be performed using a high amount of blood sample.

In general, blood contains various types of salt. A high concentration of salt particularly exists in urine and blood. Hereinafter, the term "ionized material" refers to salt ions since salt is dissolved in a solution and ionized thereafter. When salt in the blood is removed by electrodialysis, red blood cells (RBCs), which are the main parts of PCR inhibitors, are destroyed due to osmotic pressure. That is, electrodialysis of a blood sample results in the removal of salt from said sample, which leads to the osmotic lysis of RBCs, and thus salt allows for the removal of most of the PCR inhibitors such as salt and blood cells. Thus, the whole blood can be used as a PCR sample, and this effect results in an improvement in PCR efficiency to a great extent.

It usually takes a long time to dialyze a body fluid using the conventional dialysis method. In the present embodiment, instead of the simple conventional dialysis of the blood, the blood is electrically dialyzed by applying an electric field to the blood. This electrodialysis can shorten a dialysis time of the blood.

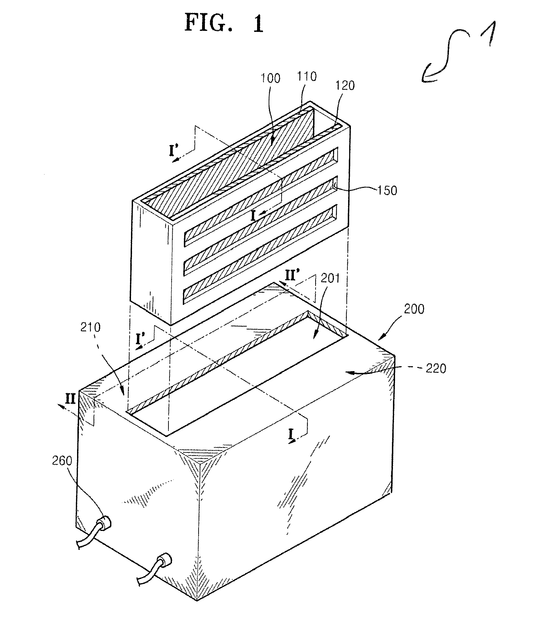

FIG. 1 illustrates a sample chamber 100 according to an embodiment of electrodialysis apparatus 1 of the present invention. The sample chamber 100 is filled with a sample, and a first and a second dialysis membrane, 110 and 120 respectively, constitute two outer walls of the sample chamber 100, which outer walls are positioned opposing each other. The sample chamber 100 is formed in a tetragonal shape, wherein the two dialysis membranes 110 and 120 form the large sidewalls of the tetragonal chamber 100. The sample chamber 100 is suited to be installed to and inserted into in a housing 200 through a slot-shaped fitting aperture 201 of the housing 200. In the assembled state of the electrodialysis apparatus 1, the sample chamber 100 with the dialysis membranes 110 and 120 is positioned in the interior of the housing 200 so that the sample chamber 100 in the housing 200 contains a sample ready to be electrodialysed. When the electrodialysis of the sample is completed, the sample chamber 100 with the dialyzed sample is detached and separated from the housing 200. The housing 200 preferably includes a slot 201 as a fitting aperture for the attachment and the detachment of the sample chamber 100 in and from the housing 200, respectively. Also, dialysis windows 150 are preferably provided on both sides of the sample chamber 100, where the first dialysis membrane 110 and the second dialysis membrane 120 are positioned. The dialysis windows 150 are openings in the chamber wall, which openings are covered by the dialysis membranes 110 and 120 to allow the first and second dialysis membranes 110 and 120 to face and be in direct contact with respective chamber solutions that may be filled in the housing 200, or respective an anode chamber 210 and a cathode chamber 220 (only indicated by arrows in Fig. 1) of the housing 200. Bubbles are also generated within the chamber solutions, and the bubbles mix and cause the chamber solutions to flow, so that electrodialysis efficiency can be improved. For this effect, air supply openings 260 are formed, which end respectively in the housing 200 or the anode chamber 210 and the cathode chamber 220 of the housing, in order to provide air inside the housing 200, said air is capable of mixing the chamber solutions.

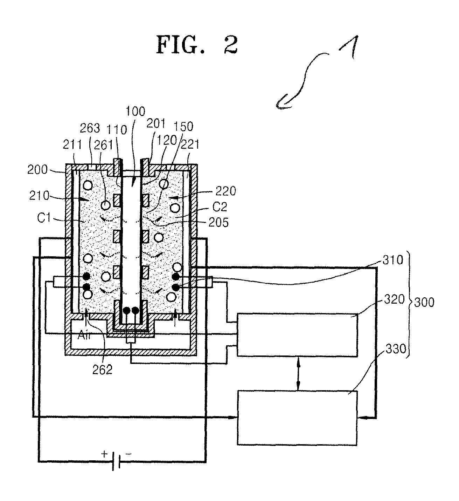

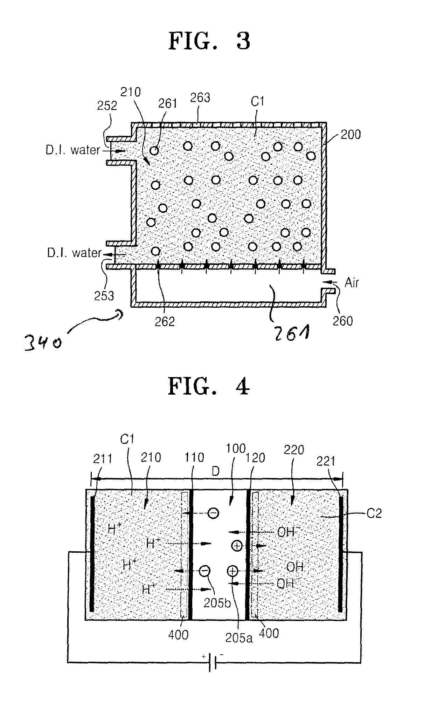

FIG. 2 illustrates the electrodialysis apparatus 1 comprising the sample chamber 100 and the housing 200 illustrated in FIG. 1, taken along a line I-I'. FIG. 3 illustrates the electrodialysis apparatus 1 comprising the sample chamber 100 and the housing 200 illustrated in FIG. 2, taken along a line II-II' illustrated in FIG. 1. Referring to FIGS. 2 and 3, the electrodialysis apparatus 1 according to the present embodiment includes the sample chamber 100, the housing 200, a control means 300 and a mixing means (340), which will now be described in detail.

The housing 200 is an outer part of the electrodialysis apparatus 1. An anode chamber 210 and a cathode chamber 220 are formed inside the housing 200 on both sides of the sample chamber 100. In the assembled state of the electrodialysis apparatus 1 of Fig. 2 and 3, the anode chamber 210 is separated from the mounted sample chamber 100 by means of the first dialysis membrane 110, and the cathode chamber 220 is separated from the mounted sample chamber 100 by means of the second dialysis membrane 120.

An anode 211 is placed inside the anode chamber 210, and a first chamber or electrode solution C1 fills the anode chamber 210. The first chamber solution C1 faces, but is separated from the sample by having the first dialysis membrane 110 there between. That is, the first chamber solution C1 is a media connecting the anode 211 of the anode chamber 210 with the sample via the first dialysis membrane 110 of the sample chamber 100. A cathode 221 is placed inside the cathode chamber 220, and a second chamber or electrode solution C2 fills the cathode chamber 220. The second chamber solution C2 faces, but is separated from the sample by having the second dialysis membrane 120 there between. That is, the second chamber solution C2 is a media connecting the cathode 221 of the cathode chamber 220 with the sample via the second dialysis membrane 120 of the sample chamber 100. Although the first and second chamber solutions C1 and C2 may have different compositions, the first and second chamber solutions preferably have substantially the same composition. It is more preferable to use deionized water as chamber solutions C1 and C2 to improve the electrodialysis efficiency. When a positive voltage is applied to the anode 211, and a negative voltage is applied to the cathode 221, an electric field is generated. Due to the electric field, ionic materials 205 of the sample move to the anode chamber 210 and to the cathode chamber 220, depending on the charge of the ions 205, resulting in electrodialysis of salt in the sample. The electric field generated when applying a voltage to the anode 211 and the cathode 221, is substantially directed orthogonally with respect to the plane of the first dialysis membrane 110 and the second dialysis membrane 120.

FIG. 4 is a diagram illustrating an electrically dialyzed state of the first and second chamber solutions C1 and C2 as the ionic materials 205 of the sample move to the first and second chamber solutions C1 and C2. That is, FIG. 4 illustrates the ion 205 transfer from a sample into the chamber solutions C1 and C2 occurring when performing electrodialysis in the electrodialysis apparatus 1. The first chamber solution C1, the first dialysis membrane 110, the sample, the second dialysis membrane 120, and the second chamber solution C2 are sequentially arranged between the anode 211 and the cathode 221, which electrodes 211 and 221 generate an electric field. Due to the electric field, the ionic materials 205 of the sample are electrically dialyzed, this is, move in the direction of the respective counter-electrode 211 or 221 through the respective first or second dialysis membrane 110, 120 into the first and second chamber solutions C1 and C2. Positively charged ionic materials 205A move through the second dialysis membrane 120 to the cathode 221 to which a negative voltage is applied, while negatively charged ionic materials 205B move through the first dialysis membrane 110 to the anode 211 to which a positive voltage is applied.

When the ionic materials 205 move to the first and second chamber solutions C1 and C2, an initial state of deionized water in each of the first and second chamber solutions C1 and C2 is changed to an ionized state. As the sample is electrically dialyzed, the amounts of hydrogen ions (H⁺) and hydrogen oxide ions (OH⁻) in the first chamber solution C1 and the second chamber solution C2, respectively, increase. When the ion concentration of each of the first and second chamber solutions C1 and C2 containing the hydrogen ions (H⁺) and hydrogen oxide ions (OH⁻) surpasses the ionic concentration of the sample, the ionic materials 205 move back from the chamber solutions C1 and C2 into the sample, and thus, the ionic concentration of the sample may increase. An ion exchange membrane that selectively blocks the movement of positive and negative ions may be used as the first and second dialysis membranes 110 and 120 in order to prevent the increase in the ionic concentration of the sample. However, the ion exchange membrane is expensive.

In an embodiment of the present invention, a molecular weight cutoff membrane, which is cheaper than the ion exchange membrane, is used as the first and second dialysis membranes 110 and 120, and the control means or control block 300 is used to maintain the ionic concentration of each of the first and second chamber solutions C1 and C2 lower than a reference ionic concentration. The molecular weight cutoff membrane is selectively transmissible for a material having a molecular weight less than a certain cutoff molecular weight. Since the molecular weight cutoff membrane is well known in the art, a detailed description thereof will be omitted. Examples of the molecular weight cutoff membrane are a cellophane membrane, a cellulose membrane, a polyether sulfone (PES) membrane, a polysulfone (PS) membrane, and a polyvinylidene fluoride (PVDF) membrane. If the molecules that are to be electrically dialyzed are ions, the ionic materials 205 can pass through the molecular weight cutoff membrane more easily than the ion exchange membrane since the molecular weight cutoff membrane does not rely on the polarity of an ion.

The ionic materials 205 that move out of the sample cause the ionic concentrations of the first and second chamber solutions C1 and C2 to increase. Thus, the concentration difference between the chamber solutions C1 and C2 and the sample over the first and second dialysis membranes 110 and 120 reduces, and electrodialysis efficiency decreases. Since lots of heat is generated due to the acceleration of the electrodialysis of the first and second chamber solutions C1 and C2, the first and second chamber solutions C1 and C2 or the sample are likely to be thermally destroyed. Thus, the control block 300 is provided so as to control and overcome this disadvantage. The control block 300 controls the ionic concentrations of the anode chamber solution C1 and the cathode chamber solution C2 to be lower than a reference ionic concentration. Further, the control means 300 is capable of removing the heat generated in the chamber solutions C1, C2 and the sample.

The amount of ions contained inside the anode chamber 210 and the cathode chamber 220 increases exponentially during electrodialysis. Hence, when the ionic concentration of each of the first and second chamber solutions C1 and C2 exceeds the reference ionic concentration, it is preferable to replace the corresponding first or second chamber solution C1 or C2 with a new chamber solution. The reference ionic concentration is preferably set to be lower than the ionic concentration of the sample in the sample chamber 100 in order to avoid that ions reenter the sample form the chamber solutions C1 and C2. That is, the reference ionic concentration that is a preset critical value used for controlling the exchange of chamber solutions is preferably lower than that of the sample. The control block 300 determines the ionic concentration in the first or second chamber solution C1 or C2 and replaces the first or second chamber solution C1 or C2 with a new chamber solution when the ionic concentration of the chamber solution C1 or C2 is equal to or higher than the reference ionic concentration.

In an embodiment of the present invention, the control block 300 determines the ionic concentration of the first or second chamber solution C1 or C2 or the sample by measuring the electrical resistance or electrical conductivity of the first chamber solution C1, the second chamber solution C2 or the sample. Therefore, the control block 300 includes a sensor unit 310, a control unit 320 and a pump 330. The sensor unit 310 is capable of sensing and determining the ionic concentration of a solution. The pump 330 supplies and discharges the first and second chamber solutions C1 and C2. The control unit 320 controls the operation of the sensor unit 310 and the pump 330. The sensor unit 310 is placed inside of each of the anode chamber 210, the cathode chamber 220 and the sample chamber 100. The sensor unit 310 measures electrical resistance or electrical conductivity of each of the first and second chamber solutions C1 and C2, and the sample, in order to thereby estimate the ionic concentrations thereof.

As illustrated in FIG. 4, dialysis interference layers 400 are formed at the respective boundaries between the first dialysis membrane 110 and the first chamber solution C1 and between the second dialysis membrane 120 and the second chamber solution C2. The density of the ionic materials 205 moving out of the sample is high at the dialysis interference layers 400 that is the interference layers 400 represent a region in the chamber solutions C1 and C2, where the ionic concentration is higher than the ionic concentration in the bulk of the chamber solutions C1 and C2. Since the interference layers 400 are formed at the boundary between the first dialysis membrane 110 and the first chamber solution C1 and between the second dialysis membrane 120 and the second chamber solution C2, respectively, the interference layers 400 adversely affect the electrodialysis.

Thus, a mixing block (340) is preferably provided to remove the dialysis interference layers 400. The mixing block generates bubbles 261 (refer to FIGS. 2 and 3) in order to remove the dialysis interference layers 400 by mixing the first and second chamber solutions C1 and C2. As a result, an ion diffusion rate and exchange rate increase.

Assuming that a stirrer that mixes the first and second chamber solutions C1 and C2 is installed according to an embodiment of the present invention, a spacing distance D between the anode 211 and the cathode 221 may increase due to the space required for the installation of the stirrer. However, the generation of the bubbles 261, which cause the mixing of respectively the first and second chamber solutions C1 and C2 does only requires minimal space for, e.g. holes in the bottom of the anode and cathode chamber 210 and 220. Hence, the spacing distance D between the anode 211 and the cathode 221 can be minimized and consequently the intensity of the electric field with respect to substantially the same voltage level can be maximized. As a result, the electrodialysis efficiency can be improved. Consequently, the anode 211 and the cathode 221 are placed in such a manner that the spacing distance D is within a range that allows the first and second chamber solutions C1 and C2 to move easily around the respective first and the second dialysis membranes 110 and 210.

FIG. 3 illustrates a sectional view of the anode chamber 210. Although not illustrated, the cathode chamber 220 has substantially the same configuration as the anode chamber 210.

In an embodiment of the control block 300, the housing 200 includes a chamber solution inlet 252 and a chamber solution outlet 253. The chamber solution inlet 252 supplies the first chamber solution C1 to the anode chamber 210, and the chamber solution outlet 253 discharges the first chamber solution C1 whose ionic concentration increases to the outside. The control unit 320 (referring to FIG. 2) controls the driving of the pump 330 based on the electrical resistance or electrical conductivity of the target solution measured by the sensor unit 310 so as to supply and discharge the chamber solution (e.g., the first chamber solution C1).

In an embodiment of the mixing block (340), the housing 200 includes an air supply opening 260 (only one opening is illustrated in FIG. 3), an air supply chamber 261, an air inlet 262, and an air outlet 263. Through the air supply opening 260, air may be directed into the air supply chamber. The air inlet 262 is designed as a passage hole in the bottom of the anode chamber 210, which bottom separated the anode chamber 210 and the air supply chamber 261. The air inlet 262 supplies the air in form of bubbles 261 from the air supply chamber 261 to the first chamber solution C1. Buoyancy ascends the air bubble in the chamber solution C2, and the air outlet 263 discharges the bubbles 261 from the chamber solution C1 to the outside.

Hereinafter, an electrodialysis method using the above described electrodialysis apparatus according to an embodiment of the present invention will be described in detail. The electrodialysis method includes arranging the first chamber solution C1, the first dialysis membrane 110, the sample, the second dialysis membrane 120, and the second chamber solution C2 in sequential order between the anode 211 and the cathode 221 that generate an electric field; and dialyzing the ionic materials 205 contained in the sample due to the electric field into the first and second chamber solutions C1 and C2. A molecular weight cutoff membrane may be used as the first and second dialysis membranes 110 and 120. The first chamber solution C1 is a chamber solution filling the anode chamber 210, while the second chamber solution C2 is a chamber solution filling the cathode chamber 220.

The sample chamber 100 includes the first and second dialysis membranes 110 and 120, and is designed in the form of an integral cartridge type so that the sample may be placed or displaced in between the first chamber solution C1 and the second chamber solution C2 through the slot 201 in the housing 200. In other words, the sample chamber 100 is attachable to or detachable from the housing 200.

The control block 300 replaces the first chamber solution C1 with new chamber solution when the ionic concentration of the first chamber solution C1 is higher than a reference ionic concentration. Similarly, the control block 300 replaces the second chamber solution C2 with new chamber solution when the ionic concentration of the second chamber solution C2 is higher than the reference ionic concentration. When both the first and second chamber solutions C1 and C2 have the ionic concentrations higher than the reference ionic concentration, the first and second chamber solutions C1 and C2 are replaced with new chamber solutions.

An experimental example of the electrodialysis method using the electrodialysis apparatus illustrated in FIGS. 1 through 4 will now be described in detail.

As a comparison group to a test group according to an embodiment of the present invention, approximately 10 ml of a sample solution was put into approximately 2 L of deionized water, and a magnetic bar was rotated to make the deionized water flow. The deionized water was replaced with new deionized water every hour, and the salt removal rate with respect to the sample was measured by determining the salt concentration in the deionized water removed with respect to the salt concentration in the initial sample. In the control experiment, an electric field was not applied, and dialysis based only on a concentration difference between the sample concentration and the deionized water was executed. After the experiment, the salt removal rates were approximately 4.8 %, 25.6 %, 38.3 %, 55 %, and 90.2 % after the elapse of approximately 5 minutes, 30 minutes, 60 minutes, 120 minutes, and 720 minutes, respectively.

In a first experimental embodiment of the present invention, a voltage was applied to the anode 211 and the cathode 221 generating an electric field, but the first and second chamber solutions C1 and C2 were not replaced with new ones during electrodialysis. The amount of the sample of the test group was approximately 10 ml, and a voltage applied to both the anode 211 and the cathode 221 was approximately 30 V. Cellophane membranes were used as the first and second dialysis membranes 110 and 120, and deionized water was used as the first and second chamber solutions C1 and C2. The amount of each of the first and second chamber solutions C1 and C2 was approximately 80 ml. Since the control block 300 was not used in this first experimental embodiment, the first and second chamber solutions C1 and C2 were not replaced with new solutions regardless of the ionic concentration of the chamber solutions C1 and C2. According to the first experiment, the salt removal rates were measured as being approximately 24.7 %, 30.2 %, and 34.4 % after the elapse of approximately 1 minute, 2 minutes, and 3 minutes, respectively. The salt removal rate of the test group sample subjected to approximately 3 minutes of the electrodialysis (e.g., approximately 34.4 %) was substantially the same as that of the comparison group sample subjected to approximately 60 minutes of the conventional dialysis (e.g., approximately 38.8 %). As compared with the amount of deionized water used in the conventional dialysis of the control (e.g., approximately 2 L), approximately 160 ml of the deionized water was used for the electrodialysis according to the first experimental embodiment. However, when the electrodialysis was executed for more than 3 minutes, the temperature of the first and second chamber solutions C1 and C2 increased indicating an equilibrium of the salt concentrations in the sample and the chamber solutions, which is accompanied with a backflow of the ionic materials 205 from the chamber solutions C1 and C2 into the sample.

In a second experiment, the electric field was applied to the anode 211 and the cathode 221, and the first and second chamber solutions C1 and C2 were replaced with new ones. The rest of the experimental conditions were substantially the same as those of the first experiment. When the first and second chamber solutions C1 and C2 contained an increasing amount of the ionic materials 205, the first and second chamber solutions C1 and C2 had decreasing levels of electrical resistance and increasing levels of electrical conductivity. In the second experimental embodiment, if the sensor unit 310 measured an electrical resistance of approximately 750 Ω or less in both of the first and second chamber solutions C1 and C2, the first and second chamber solutions C1 and C2 were replaced with new ones. If the first and second chamber solutions C1 and C2 were replaced with the new ones, since each of the anode chamber 210 and the cathode chamber 220 had an ionic concentration higher than a reference ionic concentration, the reference ionic concentration may be an ionic concentration corresponding to an electrical resistance of approximately 750 Ω. According to the second experiment, the salt removal rate with respect to the sample increased to approximately 53 % after approximately 3 minutes of electrodialysis.

In a third experiment, the electric field was applied to the anode 211 and the cathode 221, the first and second chamber solutions C1 and C2 were replaced with new ones, and bubbles 261 were supplied inside the first and second chamber solutions C1 and C2. The rest of the experimental conditions were substantially the same as those of the second exemplary experiment. The salt removal rate with respect to the sample increased to approximately 75.5 % after approximately 3 minutes of electrodialysis.

In detail, the salt removal rates at the same voltage level and electrodialysis time were approximately 34.4 % when the electric field was simply applied to the anode 211 and the cathode 221, approximately 53 % when the first and second chamber solutions C1 and C2 were replaced with new ones using the additionally provided control block 300, and approximately 75.5 % when the dialysis interference layers 400 were removed using the additionally provided mixing block.

In a fourth experiment, the experimental conditions were substantially the same as those described in the third experimental embodiment. However, a voltage of approximately 50 V was applied to the anode 211 and the cathode 221 for an elongated period of time (e.g., approximately 5 minutes). Also, when the first and second chamber solutions C1 and C2 had an electrical resistance of approximately 1,666 Ω or less, the first and second chamber solution C1 and C2 were replaced with new ones. In the fourth experiment, the reference ionic concentration became an ionic concentration corresponding to an electrical resistance of approximately 1,666 Ω. As compared with the third experiment, the fourth experiment was set in conditions in which the intensity of the electric filed increased, the voltage application time increased, and the reference ionic concentration decreased. That is, these conditions existed whereby the first and second chamber solutions C1 and C2 were replaced with new ones. In the case that urine was used as the sample in the fourth experimental embodiment, approximately 99.9 % of salt could be removed within approximately 5 minutes of electrodialysis. In the case that whole blood was used as the sample, approximately 80 % of salt could be removed within approximately 5 minutes of electrodialysis. Although the replacement of both the first and second chamber solutions C1 and C2 with new chamber solutions was described in the second through fourth experiment, one of the first and second chamber solutions C1 and C2 may be replaced with a new chamber solution when the above mentioned conditions are satisfied.

According to the above exemplary embodiments of the present invention, the electrodialysis apparatus and the electrodialysis method using the same provide several effects as follows.

First, since the electrodialysis takes place using the molecular weight cutoff membrane, dialysis efficiency can be improved compared with when using the conventional dialysis membrane (e.g., an ion exchange membrane). Also, whole blood that is not diluted can be used as a PCR sample after performing electrodialysis according to the present invention.

Second, the control block allows the replacement of the chamber solution with a new one before the chamber solution reaches a certain concentration. Thus, a diffusion rate based on an ionic concentration gradient increases, and this increasing diffusion rate makes it possible to remove salt contained in the sample within a short period of time. Since the amounts of hydrogen ions (H⁺) and hydrogen oxide ions (OH⁻) that are generated within the anode and cathode chambers can be reduced, it becomes unnecessary to use an expensive ion exchange membrane for the reduction of these ions. Also, salt removal efficiency can be improved since an ionic concentration gradient can be maximized.

Third, the mixing block that generates bubbles is used to increase the flux of the solutions around the dialysis membranes and the interfacial area. As a result, the thickness of the dialysis interference layers formed at the interfaces of the dialysis membranes decreases and the diffusion rate increases, so that an ion exchange rate can increase. Since the distance between the electrodes is shortened, and simultaneously the mixing efficiency increases, the intensity of an electric field can increase substantially at the same voltage level.

Fourth, one or both of the chamber solutions can be replaced with a new one or with new ones by measuring electrical resistances or electrical conductivities of the anode chamber, the cathode chamber, and the sample chamber. That is, a minimum amount of each of the chamber solutions can be replaced during an optimal replacement period, and thus, the amount of chamber solution used can be reduced. Also, it is possible to prevent ions generated by the electrodialysis from flowing back into the sample.

Fifth, the individual chamber solutions are not replaced partially but entirely with a new one, and thus, electrodialyzed materials whose amount increases exponentially can be removed concurrently. As a result, an ionic concentration gradient can be maximized. Also, since the electrodialysis of the chamber solutions can be suppressed, heat generation occurring during the electrodialysis can be reduced. Thus, electrodialysis efficiency and sample damage can be reduced.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. An electrodialysis apparatus (1) comprising:
a sample chamber (100);
a first dialysis membrane (110) and a second dialysis membrane (120);
an anode chamber (210) including an anode (211), wherein the first dialysis membrane (110) separates the anode chamber (210) and the sample chamber (100); and
a cathode chamber (220) including a cathode (221), wherein the second dialysis membrane (120) separates the cathode chamber (220) and the sample chamber (100).

2. The electrodialysis apparatus (1) of claim 1, wherein the first dialysis membrane (110) and the second dialysis membrane (120) form walls of the sample chamber (100).

3. The electrodialysis apparatus (1) of claim 2, wherein the first dialysis membrane (110) and the second dialysis membrane (120) form walls, which are positioned opposing each other.

4. The electrodialysis apparatus (1) of any of claims 1 to 3, wherein at least one of the first dialysis membrane (110) and the second dialysis membrane (120) is a molecular weight cutoff membrane.

5. The electrodialysis apparatus (1) of claim 4, wherein the molecular weight cutoff membrane includes one selected from a cellophane membrane, a cellulose membrane, a PES (polyether sulfone) membrane, a PS (polysulfone) membrane, a PVDF (polyvinylidene fluoride) membrane, and a combination thereof.

6. The electrodialysis apparatus (1) of any of claims 1 to 5, further comprising a control means (300) capable of controlling an ionic concentration of a chamber solution (C1, C2) that is present in the anode chamber (210) and/or the cathode chamber (220) to be lower than a reference ionic concentration.

7. The electrodialysis apparatus (1) of claim 6, wherein the control means (300) comprises means for replacing the whole or a part of the chamber solutions (C1, C2) with new chamber solutions.

8. The electrodialysis apparatus (1) of claim 7, wherein the control means (300) comprises:
a sensor unit (310) for sensing the ionic concentration of the chamber solution (C1, C2) in the anode chamber (210), the cathode chamber (220) and/or a sample solution in the sample chamber; and
a pump (330) for supplying or discharging the chamber solutions.

9. The electrodialysis apparatus (1) of claim 8, wherein the sensor unit (310) comprises a electrical resistance measuring means or a electrical conductivity measuring means capable of sensing the ionic concentrations of a chamber solution (C1, C2) in the anode chamber, the cathode chamber and/or a sample solution in the sample chamber.

10. The electrodialysis apparatus (1) of any of claims 1 to 9, further comprising a mixing means (340) capable of mixing the chamber solutions in the anode chamber (210) and/or the cathode chamber (220).

11. The electrodialysis apparatus (1) of claim 10, wherein the mixing means (340) comprises means (262) capable of supplying bubbles into the anode chamber (210) and/or the cathode chamber (220).

12. The electrodialysis apparatus (1) of claim 11, wherein the anode (211) and the cathode (221) are spaced apart from each other by a range of minimum distance (D) so as to allow smooth flux of the chamber solutions (C1, C2) around the respective first and second dialysis membranes (110, 120).

13. The electrodialysis apparatus (1) of any of claims 1 to 12, wherein the sample chamber (100) is formed in a tetragonal shape.

14. The electrodialysis apparatus (1) of claim 13, wherein the first dialysis membrane (110) and the second dialysis membrane (120) constitute, at least section wise, side wall areas of the sample chamber (100).

15. The electrodialysis apparatus (1) of claim 2, wherein the sample chamber (100) is attachable to or detachable from the electrodialysis apparatus (1).

16. The electrodialysis apparatus (1) of any of claims 1 to 15, further comprising:
a housing (200) capable of being filled with a chamber solution, wherein
the anode (211) and the cathode (221) are positioned spaced apart from each other inside the housing (200).

17. The electrodialysis apparatus (1) of claims 15 and 16, wherein the sample chamber (100) is attachable to or detachable from the housing (200) in a position between the anode (211) and the cathode (221).

18. An electrodialysis apparatus (1) of claim 1,
wherein the chamber solution (C1) in the anode chamber (210), the first dialysis membrane (110), the sample in the sample chamber (100), the second dialysis membrane (120), and the chamber solution (C2) in the cathode chamber (220) are arranged in sequential order between the anode (211) and the cathode (221).

19. The electrodialysis apparatus (1) of claim 11, wherein the means (262) capable of supplying bubbles comprises at least one outlet (262) through which, during operation, bubbles are released into the anode chamber (210) and/or the cathode chamber (220), wherein the at least one outlet (262) is positioned such that to the bubbles released through said outlet (262) are capable of removing a dialysis interference layer (400) formed at the interface between the first dialysis membrane (110) and the anode chamber (210) and/or between the second dialysis membrane (120) and the cathode chamber (220).

20. An electrodialysis method comprising:
arranging a first chamber solution (C1), a first dialysis membrane (110), a sample, a second dialysis membrane (120), and a second chamber solution (C2) in sequential order between an anode (211) and a cathode (221) that are generating an electric field; and
dialyzing ionic materials contained in the sample through the respective dialysis membrane (110, 120) into the first and second chamber solutions (C1, C2) based on the electric field.

21. The electrodialysis method of claim 20,
wherein the at least one of the first dialysis membrane (110) and the second dialysis membrane (120) is a molecular weight cutoff membrane.

22. The electrodialysis method of claim 20 or 21, wherein the chamber solutions (C1, C2) include deionized water.

23. The electrodialysis method of any of claims 20 to 22, further comprising the step of controlling the ionic concentration of the first chamber solution (C1) and/or the second chamber solution (C2) to be lower than a reference ionic concentration.

24. The electrodialysis method of claim 23, wherein the whole or a part of the first chamber solution (C1) and/or the second chamber solution (C2) is replaced with new chamber solution if the ionic concentration is higher than the reference ionic concentration.

25. The electrodialysis method of any of claims 20 to 24, wherein the reference ionic concentration is lower than an ionic concentration of the sample chamber.

26. The electrodialysis method of any of claims 20 to 25, wherein electrical resistance or electrical conductivity of the first chamber solution (C1), the second chamber solution (C2) and/or the sample solution is measured.

27. The electrodialysis method of any of claims 20 to 26, further comprises the step of mixing the first chamber solution (C2) and/or the second chamber solution (C2).

28. The electrodialysis method of claim 27, comprising releasing bubbles into the first chamber solution (C1) and/or the second chamber solution (C2).

29. The electrodialysis method of any of claims 20 to 28, further comprising removing a dialysis interference layer (400) formed at an interfaces between the first dialysis membrane (110) and the first chamber solution (C1) and/or between the second dialysis membrane (120) and the second chamber solution (C2).

30. The electrodialysis method of claims 28 and 29, comprising removing a dialysis interference layer (400) by means of bubbles.

31. The electrodialysis method of claim 29 or 30, wherein the anode (211) and the cathode (221) are spaced a minimum distance (D) from each other in order to maximize the intensity of the electric field.

32. The electrodialysis method of any of claims 20 to 29, wherein the sample is filled between the first dialysis membrane (110) and the second dialysis membrane (120) of a sample chamber (100) designed in the form of an integral cartridge type, and attaching the sample cartridge to a space between the first chamber solution (C1) and the second chamber solution (C2); applying a voltage to the anode (211) and the cathode (221) and performing electrodialysis; and detaching the sample cartridge containing the electrodialysed sample from the space between the first chamber solution (C1) and the second chamber solution (C2).
